(19) Europäisches Patentamt
European Patent Office
Office européen des brevets

(11) Numéro de publication: **0 189 336**
**A1**

(12) **DEMANDE DE BREVET EUROPEEN**

(21) Numéro de dépôt: **86400015.3**

(22) Date de dépôt: **07.01.86**

(51) Int. Cl.⁴: **A 61 K 45/06**, A 61 K 31/55, A 61 K 31/505 // (A61K31/55, 31:505),(A61K31/55, 31:445),(A61K31/505, 31:44), (A61K31/505, 31:275)

(30) Priorité: **18.01.85 FR 8500678**
**26.02.85 FR 8502725**
**26.02.85 FR 8502726**

(43) Date de publication de la demande: **30.07.86**
**Bulletin 86/31**

(84) Etats contractants désignés: **BE CH DE FR GB IT LI LU NL SE**

(71) Demandeur: **SYNTHELABO, 58, rue de la Glacière, F-75621 Paris Cedex 13 (FR)**

(72) Inventeur: **Cavero, Icilio, 8, rue Gabriel Fauré, F-94000 Creteil (FR)**
Inventeur: **Cazor, Jean-Louis, 108, rue de la Folie Méricourt, F-75011 Paris (FR)**
Inventeur: **Hicks, Peter, 14, rue des Amandiers, F-94230 Cachan (FR)**
Inventeur: **Langer, Salomon, 92, rue Jouffroy, F-75017 Paris (FR)**

(74) Mandataire: **Thouret-Lemaitre, Elisabeth et al, Service Brevets - SYNTHELABO 58, rue de la Glacière, F-75621 Paris Cedex 13 (FR)**

(54) **Compositions pharmaceutiques contenant un alpha-bloquant et un antagoniste du calcium.**

(57) Compositions pharmaceutiques contenant, à titre de substance active, une association d'un α-bloquant et d'un antagoniste du calcium.

Application en thérapeutique.

EP 0 189 336 A1

La présente invention a pour objet des compositions pharmaceutiques contenant un α-bloquant et un antagoniste du calcium, compositions pharmaceutiques destinées au traitement de maladies cardiovasculaires, plus particulièrement de l'hypertension quelle que soit son origine et l'angine de poitrine.

La demanderesse a constaté que, de manière surprenante, il existe un effet de synergie important entre les propriétés antihypertensives de deux de ces composés, α-bloquants et antagonistes du calcium, lorsque l'on les associe.

La demanderesse a déjà decrit et revendiqué dans un brevet précédent l'association alfusozine/diltiazem.

Les α-bloquants possédant des propriétés antihypertensives et plus particulièrement appropriés selon l'invention sont l'alfuzosine, la prazosine, la térazosine, la doxazosine, la trimazosine, la bunazosine, l'urapidil et l'indoramine dont les formules 1, 2, 3, 4, 5, 6, 7 et 8 sont données dans l'annexe 1.

Les antagonistes du calcium appropriés selon l'invention sont le diltiazem, la nifédipine et le vérapamil dont les formules 9, 10 et 11 sont données dans l'annexe 2.

Les compositions pharmaceutiques de l'invention ont été soumises à une série d'essais pharmacologiques qui révèlent leurs intéressantes propriétés dans le domaine cardiovasculaire.

Le test utilisé est le suivant :
des rats spontanément hypertendus ("SHR") âgés de plus de 5 mois, sont placés pendant 30 mn, dans une cage à air conditionné maintenue à 28°C. La pression systolique des rats est mesurée selon la méthode décrite par Gerold et Tschirky (Arzneim. Forsch. 1968, 18, 1285).

On administre à un groupe de 6 rats SHR une dose d'antagoniste du calcium et une dose d'α-bloquant séparément ou l'association des deux doses d'α-bloquant et d'antagoniste du calcium.

La pression systolique est mesurée avant l'administration des agents thérapeutiques puis 3 à 5 heures après l'administration d'un des composés ou de l'association de deux des agents thérapeutiques.

Les résultats sont donnés sous forme de diminutions de la pression sanguine par rapport à la pression de base.

Dans le tableau suivant sont donnés les résultats obtenus, pour les composés administrés seuls, et pour les associations de deux des agents thérapeutiques.

Tableau

| Composé | dose mg/kg p.o. | pression de base (mm Hg) | Diminution de la pression systolique (mm Hg) |
|---|---|---|---|
| diltiazem | 12.5 | 222 ± 4 | -1 ± 2 |
| prazosine | 0.3 | 215 ± 4 | -26 ± 5 |
| prazosine + diltiazem | 0.3+12.5 | 218 ± 6 | -62 ± 5* |
| trimazosine | 3.0 | 210 ± 3 | -3 ± 2 |
| trimazosine + diltiazem | 3.0+12.5 | 210 ± 4 | -44 ± 6* |
| térazosine | 0.5 | 208 ± 3 | -28 ± 4 |
| térazosine + diltiazem | 0.5+12.5 | 212 ± 5 | -45 ± 6* |
| urapidil | 3.0 | 208 ± 4 | -13 ± 7 |
| urapidil + diltiazem | 3+12.5 | 208 ± 3 | -32 ± 2* |
| vérapamil | 15 | 217 ± 5 | -28 ± 9 |
| alfuzosine | 1 | 221 ± 2 | -15 ± 5 |
| vérapamil + alfuzosine | 15+1 | 209 ± 6 | -120 ± 5* |
| nifédipine | 10 | 219 ± 4 | -5 ± 4 |
| alfuzosine | 0,3 | 213 ± 2 | 13 ± 4 |
| alfuzosine | 1 | 216 ± 2 | 18 ± 7 |
| nifédipine + alfuzosine | 10+0,3 | 211 ± 3 | -32 ± 2* |
| nifédipine + alfuzosine | 10+1 | 219 ± 3 | -55 ± 5* |

* Effet significativement supérieur à une simple addition des effets de chacun des composés.

Ces résultats montrent que l'association de l'un des antagonistes du calcium choisis selon l'invention avec un des α-bloquants cités produit un effet antihypertenseur significativement plus important que la somme des effets engendrés par chacun des composés administrés isolément.

Il existe donc une synergie entre les effets antihypertenseurs de l'antagoniste du calcium et de l'α-bloquant.

Les compositions pharmaceutiques de l'invention contiennent de 5 à 120 mg de l'un des antagonistes du calcium et de 0,1 à 30 mg de l'un des α-bloquants cités, par unité de prise.

Les compositions pharmaceutiques préférées de l'invention sont les suivantes :

- compositions contenant du vérapamil et de l'alfuzosine,
- compositions contenant de la nifédipine et de l'alfuzosine,
- compositions contenant du diltiazem et de la prazosine,
- compositions contenant du diltiazem et de la térazosine,
- compositions contenant du diltiazem et de la doxazosine,
- compositions contenant du diltiazem et de la trimazosine,
- compositions contenant du diltiazem et de la bunazosine,
- compositions contenant du diltiazem et de l'urapidil,
- compositions contenant du diltiazem et de l'indoramine.

Les compositions pharmaceutiques de l'invention peuvent être présentées sous toute forme appropriée pour l'administration par voie orale ou parentérale, en association avec tout excipient approprié.

Les compositions pharmaceutiques de l'invention peuvent être utilisées pour le traitement de l'hypertension, de l'angine de poitrine et pour le traitement des autres maladies telles que l'asthme et les affections urologiques.

La posologie quotidienne est telle que l'on administre de 5 à 240 mg de l'un des antagonistes du calcium et de 1 à 50 mg de l'un des α-bloquants.

Annexe 1

Alfuzosine

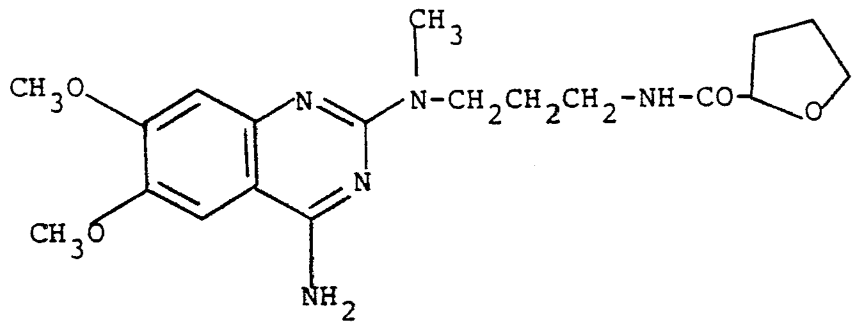

(1)

- amino-4 diméthoxy-6,7 quinazolines

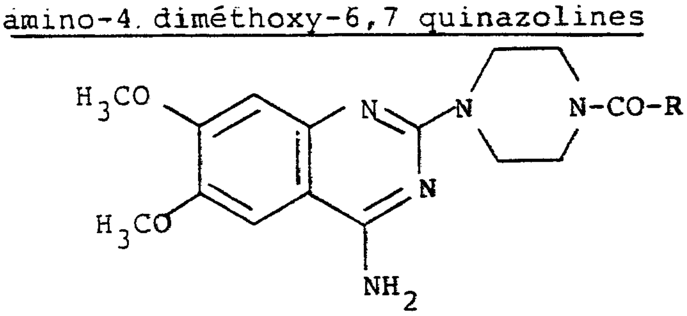

. prazosine R = 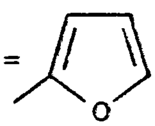 (2)

. térazosine R = 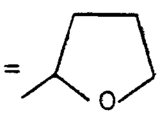 (3)

. doxazosine R = 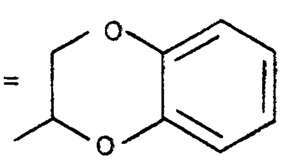 (4)

<u>Annexe 1</u> (suite et fin)

- trimazosine

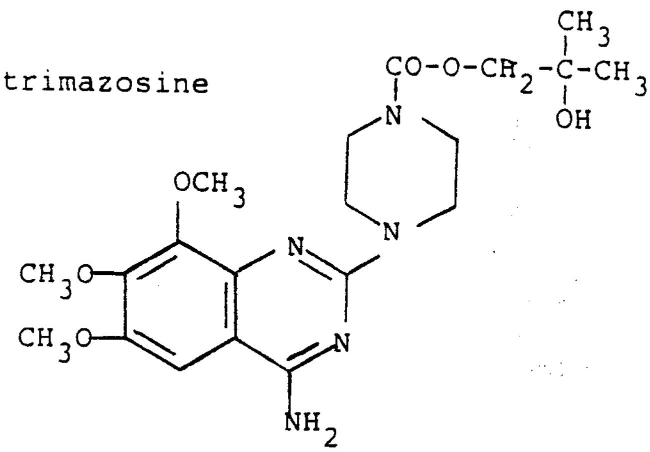

(5)

- bunazosine

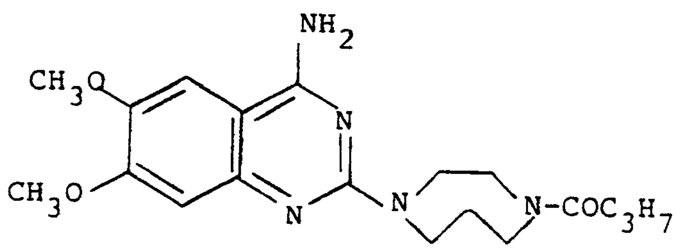

(6)

- urapidil

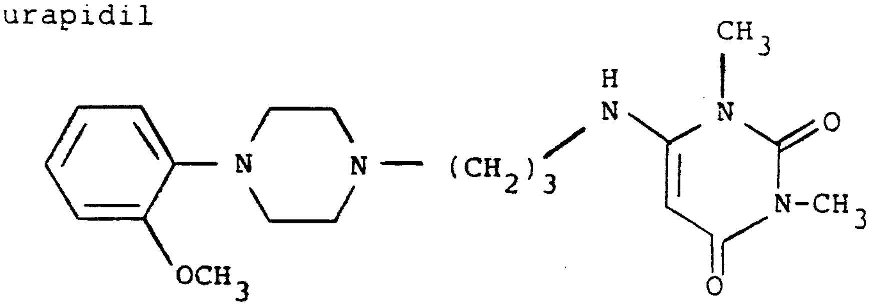

(7)

- indoramine

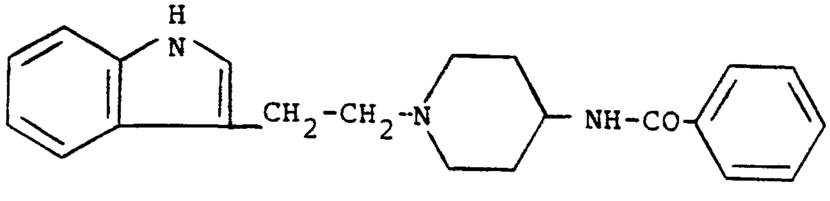

(8)

0189336

Annexe 2

- diltiazem

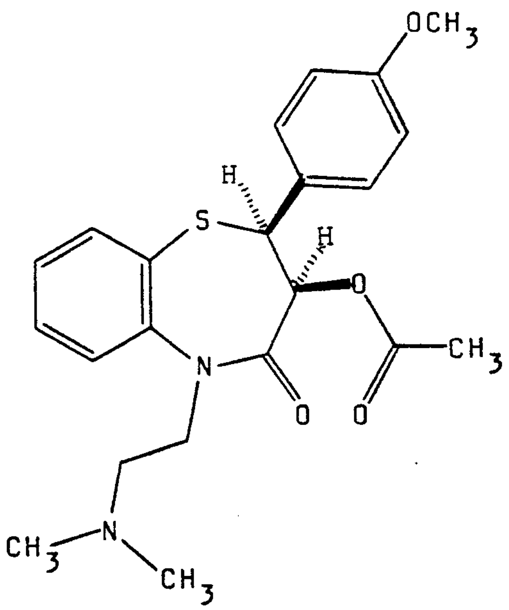

(9)

- nifédipine

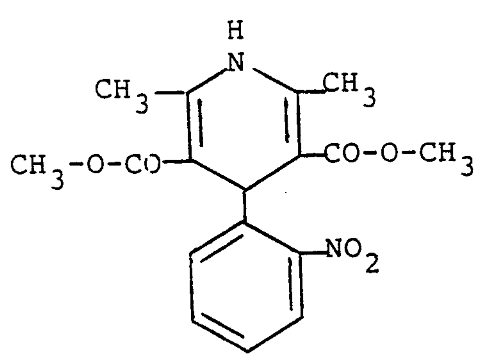

(10)

Annexe 2 (suite et fin)

Vérapamil

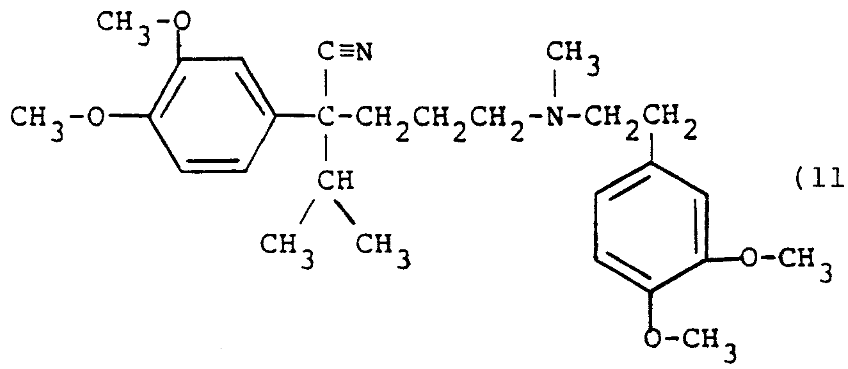

(11)

## Revendications

1. Composition pharmaceutique contenant une association d'un α-bloquant et d'un antagoniste du calcium, à l'exception de l'association alfusozine/diltiazem.

2. Composition pharmaceutique selon la revendication 1, caractérisée par le fait que l'α-bloquant est choisi parmi l'alfuzosine, la prazosine, la térazosine, la doxazosine, la trimazosine, la bunazosine, l'urapidil et l'indoramine.

3. Composition pharmaceutique selon la revendication 1 ou la revendication 2, caractérisée par le fait que l'antagoniste du calcium est choisi parmi le diltiazem, la nifédipine et le vérapamil.

4. Composition pharmaceutique contenant une association de vérapamil et d'alfuzosine.

5. Composition pharmaceutique contenant une association de nifédipine et d'alfuzosine.

6. Composition pharmaceutique contenant une association de diltiazem et de prazosine.

7. Composition pharmaceutique contenant une association de diltiazem et de térazosine.

8. Composition pharmaceutique contenant une association de diltiazem et de doxazosine.

9. Composition pharmaceutique contenant une association de diltiazem et de trimazosine.

10. Composition pharmaceutique contenant une association de diltiazem et de bunazosine.

11. Composition pharmaceutique contenant une association de diltiazem et d'urapidil.

12. Composition pharmaceutique contenant une association de diltiazem et d'indoramine.

13. Composition pharmaceutique selon l'une quelconque des revendications 1 à 12 caractérisée par le fait qu'elle contient de 5 à 240 mg de l'un des antagonistes du calcium et de 1 à 50 mg de l'un des agents α-bloquants.

Office européen des brevets

**RAPPORT DE RECHERCHE EUROPEENNE**

0189336

Numero de la demande

EP 86 40 0015

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 96, no. 25, 21 juin 1982, page 52, no. 210659e, Columbus, Ohio, US; P. THIEVANT et al.: "Effects of diltiazem on renovascular-hypertensive on normotensive rats", & GEN. PHARMACOL., 1982, 13(2), 165-8 * Abrégé * | 1-13 | A 61 K 45/06<br>A 61 K 31/55<br>A 61 K 31/505 //<br>(A 61 K 31/55<br>A 61 K 31:505)<br>(A 61 K 31/55<br>A 61 K 31:445)<br>(A 61 K 31/505<br>A 61 K 31:44 )<br>(A 61 K 31/505<br>A 61 K 31:275) |
| A | CHEMICAL ABSTRACTS, vol. 97, no. 3, 19 juillet 1982, page 51, no. 16866a, Columbus, Ohio, US; M.A. WEBER et al.: "A vasodilator that avoids renin stimulation and fluid retention: antihypertensive treatment with trimazosin", & CLIN. PHARMACOL. THER. 1982, 31(5), 572-8 * Abrégé * | 1-13 | |
| | | | DOMAINES TECHNIQUES RECHERCHES (Int. Cl.4) |
| A | CHEMICAL ABSTRACTS, vol. 100, no. 5, 30 janvier 1984, page 24, no. 29443r, Columbus, Ohio, US; J.L. REID et al.: "Pharmacokinetics and pharmacodynamics of trimazosin in man", & AM. HEART. J. 1983, 106(5, Pt. 2), 1222-8 * Abrégé * | 1-13 | A 61 K |

--- -/-

Le present rapport de recherche a été établi pour toutes les revendications

| Lieu de la recherche | Date d'achèvement de la recherche | Examinateur |
|---|---|---|
| LA HAYE | 15-04-1986 | BRINKMANN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

Office européen des brevets

**RAPPORT DE RECHERCHE EUROPEENNE**

0189336

Numéro de la demande

EP 86 40 0015



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 101, no. 11, 10 septembre 1984, page 39, no. 83745z, Columbus, Ohio, US; I. KOBRIN et al.: "Urapidil in normotensive and spontaneously hypertensive rats: the effects on systemic regional hemodynamics and cardiac mass", & J. HYPERTENS. 1984, 2(3), 317-20 * Abrégé * | 1-13 | |
| A | FR-A-2 466 462 (SYNTHELABO SA) | 1-13 | |
| A | FR-A-2 445 323 (SYNTHELABO SA) | 1-13 | |
| A | FR-A-2 421 888 (SYNTHELABO SA) | 1-13 | |
| | | | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 15-04-1986 | BRINKMANN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82